Europäisches Patentamt

(19) European Patent Office

Office européen des brevets

(11) Publication number: **0 254 693**
**A1**

(12)

# EUROPEAN PATENT APPLICATION

(21) Application number: **87830286.8**

(22) Date of filing: **24.07.87**

(51) Int. Cl.⁴: **A 61 K 31/40**
**A 61 K 9/48, A 61 K 9/66**

(30) Priority: **25.07.86 IT 2126386**

(43) Date of publication of application:
**27.01.88 Bulletin 88/04**

(84) Designated Contracting States:
**CH DE ES FR LI NL**

(71) Applicant: **MED-IMPORT INTERNATIONAL S.r.l.**
**Via Vetreria, 1**
**GRANDTE Como (IT)**

(72) Inventor: **Casero, Riccardo**
**Via Rovascino, 21**
**Lipomo (Como) (IT)**

(74) Representative: **Aimi, Luciano et al**
**c/o Società Italiana Brevetti S.p.A. Via Carducci 8**
**I-20123 Milano (IT)**

(54) Pharmaceutical form for enteric administration of etodolic acid or its salts, in admixture with excipients.

(57) The etodolic acid, an anti-inflammatory non steroidic agent, and its derivatives in admixture with excipients for suppositories, such as vegetable oils, bees wax, lecithin, etc, enclosed in hard gelatine capsules, made opaque by mineral opacifiers, are easily administrered by rectal way. The so prepared capsules allow for the preservation of the anti-inflammatory ingredient in admixture with excipients for 3-4 years without appreciable degradation of the therapeutical properties.

EP 0 254 693 A1

## Description

## "PHARMACEUTICAL FORM FOR ENTERIC ADMINISTRATION OF ETODOLIC ACID OR ITS SALTS, IN ADMIXTURE WITH EXCIPIENTS"

The present invention relates to a pharmaceutical form for the enteric administration of a non-steroidic anti-inflammatory medicine in admixture with solid and/or oleus excipients. In particular it relates to the preparation by rectal way of the etodolic acid or of its salts, particularly the sodium salt, in admixture with excipients.

It is known that some non-stereoidic compounds carrying on non-inflammatory and anti edemigenic activities show a poor stability if mixed with some excipients suitable for the administration by rectal way. It is also known that the rectal way is a particularly preferred way, as gastro-damaging properties are ascribed to such therapeutically active substances in various degrees. Among such anti-inflammatory compounds of non-stereoidic type, the etodolic acid (1.8-diethyl - 1.3.4.9 tetrahydropyrane - [3-4-6] indol-acetic acid) turned out to be unstable when mixed with the excipients generally used for preparing suppositories and ovules for rectal and vaginal administration.

The applicant has therefore carried out several studies, by employing known excipients in various combinations, with a view to possibly finding a combination adequate for the therapeutic usage in order to let unaltered the healing properties of the etodolic acid and of its salts for a sufficiently long period. Such studies and researches have not brought nevertheless to positive results for the industrial appliances, until it has been surprisingly discovered that an admixture of etodolic acid or of a salt of it (for example the sodium salt) with some vegetable oils, bees wax and lecithin, enclosed in gelatine capsules suitably specified, could be preserved, without a noticeable reduction of the therapeutic activity for a period of time of 36-48 months from the date of packing. The combination of substances and the particular packing from allow consquently for the industrial preparation of etodolic acid included in rectal capsules safely preservable for a period of time definitely longer than that of any other formulation tested so far.

According to a preferred preparatione technique, but absolutely without any restricting purposes, the etodolic acid or a salt of it is first emulsified with soyabean oil and, to the so obtained emulsion, minor amounts of other vegetable oils, hydrogenated if that be the case, such as palm oil, coconut oil and also beewax and/or lecithin. The so obtained final emulsion is then enclosed according to known methods in gelatine capsules suitably plasticized and made opaque by using mineral pigments such as titanium dioxide, zinc oxide, calcium carbonate, etc.

Some formulation examples will better make clear the nature and the significance of the present invention:

    A) Etodolic acid  6 parts
  soya bean oil  8 parts
  palm oil  2 parts
  bees wax 1  part
    B) Sodium etodolate  6 parts
  soya bean oil  6 parts
  palm oil  2 parts
  bees wax  1 part
    C) Etodolic acid  3 parts
  soya bean oil  2 parts
  hydrogenated coconut oil  2 parts
  lecithin  1 part
    D) Sodium etodolate  12 parts
  soya bean oil  12 parts
  palm oil  2.4 parts
  hydrogenated coconut oil  1.2 parts
  bees wax  1.2 parts
  lecithin  1.2 parts

Each of the above obtained mixtures is then enclosed in gelatine capsules having the following composition:

  gelatine  54-80%
  glycerol  10-36%
  water  7-15%
  titanium dioxide  0.5-5%

The so obtained gelatinous capsules lend themselves in a particular way to the rectal way administration, but can also be employed, by suitable modification of the recipe, also to the oral administration, for instance employing a soft gelatine. Consequently the weight of each individual capsule may vary depend ing on the way of administration, on the forecast daily dosage, on the patients' age, etc.

The stability of the active constituent contained into the capsules manufactured according to the present invention, under normal preservation conditions can reach and exceed a four years duration.

In order to mark the particular ways of administration or differentiated dosages, the capsules according to the present invention may contain, in addition to the above mentioned opacifiers, also some dyestuffs.

While the present invention has been described with reference to the base of some of its examples of fulfilment, it is clear that changes and/or modifications may be made by the skilled in the art without departing from the spirit and scope of the invention.

## Claims

1. A pharmaceutical form for the enteric administration of anti-inflammatory non steroidic agents in admixture with excipients, characterized in that said anti-inflammatory agents in admixture with substantially oleous excipients are enclosed in gelatine capsules opacified by inorganic pigments.

2. The pharmaceutical form according to claim 1, characterized in that said non steroidic anti-inflammatory agents are etodolic acid and its pharmaceutically acceptable salts.

3. The pharmaceutical form according to claim 2, characterized in that said substantially oleous excipients are selected from a group consisting of soya bean oil, palm oil, hydrogenated coconut oil, bees wax, lecithin and their mixtures.

4. The pharmaceutical form according to any of the claims preceding claims, characterized in that said inorganic pigments are selected from a group consisting of titanium dioxide, zinc oxide and calcium carbonate.

5. The pharmaceutical form according to claim 4, characterized in that said gelatine capsule also contains a dyestuff.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 104, no. 10, 10th March 1986, page 398, abstract no. 75071y, Columbus, Ohio, US; & JP-A-60 218 318 (S.S. PHARMACEUTICAL CO., LTD) 01-11-1985 * Abstract * | 1-5 | A 61 K 31/40<br>A 61 K 9/48<br>A 61 K 9/66 |
| | --- | | |
| Y | CHEMICAL ABSTRACTS, vol. 100, no. 22, 28th May 1984, page 321, abstract no. 180047k, Columbus, Ohio, US; M. KRAML et al.: "Bioavailability studies with etodolac in dogs and man", & BIOPHARM. DRUG DISPOS. 1984, 5(1), 63-74 * Abstract * | 1-5 | |
| | --- | | TECHNICAL FIELDS SEARCHED (Int. Cl.4) |
| Y | US-A-2 770 553 (J.F. WEIDENHEIMER) * Column 1, lines 18-21; column 2, lines 51-53,62-68 * | 1-5 | A 61 K |
| | --- | | |
| A | EP-A-0 147 146 (AMERICAN HOME PRODUCTS CORP.) * Page 10, example 3; page 13, claim 7 * | 2,3 | |
| | --- | | |
| A | DE-A-2 824 362 (J. KLOSA) * Page 6, example 1 * | 3 | |
| | ---     -/- | | |

The present search report has been drawn up for all claims

| Place of search | Date of completion of the search | Examiner |
|---|---|---|
| THE HAGUE | 21-10-1987 | FOERSTER W.K. |

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| A | US-A-4 333 957 (Y. OKAJIMA) <br> * Column 2, lines 22-31; claims 1-4 * | 4,5 | |
| | ----- | | |

TECHNICAL FIELDS SEARCHED (Int. Cl.4)

The present search report has been drawn up for all claims

| Place of search | Date of completion of the search | Examiner |
|---|---|---|
| THE HAGUE | 21-10-1987 | FOERSTER W.K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82